# EUROPEAN PATENT SPECIFICATION

(11) **EP 3 479 775 B1**
(45) Date of publication and mention of the grant of the patent: **22.03.2023**
(21) Application number: 17818869.4
(22) Date of filing: 29.03.2017
(51) Int. Cl.: A61B 17/00, A61B 34/30, B25J 9/04, B25J 18/02, B25J 18/04, B23Q 11/00, B25J 18/00, B25J 19/00, B25J 9/16, A61B 90/00, F16M 11/04, F16M 11/08, F16M 11/18, F16M 11/20

(54) **SURGICAL ROBOT AND MECHANICAL ARM THEREOF**
CHIRURGISCHER ROBOTER UND MECHANISCHEM ARM DAFÜR
ROBOT CHIRURGICAL ET SON BRAS MÉCANIQUE

(30) Priority: 29.06.2016 CN 201610496647
(43) Date of publication of application: 08.05.2019
(73) Proprietor: Shanghai Microport Medbot (Group) Co., Ltd., Shanghai 201203 (CN)
(72) Inventor: HE, Chao, Shanghai 201201 (CN); LI, Tao, Shanghai 201201 (CN); YUAN, Shuai, Shanghai 201201 (CN); WANG, Changchun, Shanghai 201201 (CN); YANG, Jie, Shanghai 201201 (CN)
(74) Representative: Patentship Patentanwaltsgesellschaft mbH
(86) International application number: PCT/CN2017/078604
(87) International publication number: WO 2018/000870

(56) References cited:
- EP-A1- 0 629 475
- WO-A1-2010/130817
- WO-A1-2013/084221
- WO-A1-2015/142786
- WO-A2-2011/097132
- CN-A- 101 487 558
- CN-A- 102 469 995
- CN-A- 103 056 646
- CN-A- 103 876 834
- CN-A- 104 942 826
- CN-A- 105 232 153
- CN-A- 106 037 934
- CN-A- 106 175 934
- JP-A- S61 820
- JP-A- H08 318 483
- JP-A- H09 285 989

## Description

### Technical Field

The present invention relates to the field of medical devices and, in particular, to a robotic arm for a medical surgical robot for use in minimally invasive surgery.

### Background

Minimally invasive surgery, an interventional procedure that provides surgical treatment based on a minimal channel and a corresponding minimally invasive instrument, achieves comparable or better efficacy compared to traditional open surgery. Minimally invasive surgery is an area of intense interest in modern medical technology because of its various advantages over traditional open surgery, such as minimal operation wound, less bleeding and rapid physical recovery.

As part of minimally invasive procedures, minimally invasive surgical robots have been growing rapidly. With patients' increasing requirements for surgical outcomes as well as physicians' increasing requirements for surgical methodologies, medical robotics are being more and more applied to the medical field, especially to minimally invasive surgery. Compared to classical minimally invasive procedures, those conducted by minimally invasive surgical robots mitigate physicians' stress during procedures and allow their more precise and flexible operations.

Different from industrial robots, minimally invasive surgical robots are imposed with more stringent requirements in terms of motion accuracy, motion space and motion mode. Difficulty in manipulation of a minimally invasive surgical robot mainly lies in preoperative positioning and intraoperative control. Preoperative positioning involves positional adjustments between the surgical robot and the patient before the start of the procedure, while intraoperative control relates to real-time operation and control during the procedure. For any surgical robot, the positional adjustments of surgical instruments are necessary to be made by its robotic arm before the start of the procedure, and such adjustments will directly affect whether the procedure can be conducted successfully.

EP 0 629 475 A1 discloses an articulated arm for manipulators and industrial robots comprising an adjusting lever which can be pivoted by motor about a fixed axle and at the other end of which an intermediate lever is pivotably articulated via a shaft supported rotatably on the adjusting lever, the linearly movable lever arm being seated at the other end of the intermediate lever on a shaft, supported rotatably on the intermediate lever, and so as to be pivotable with said shaft, and tracking transmission means dependent on the pivoting movement of the adjusting lever acting at the shaft connected so as to be fixed in terms of rotation to the intermediate lever and at the shaft connected so as to be fixed in terms of rotation to the lever arm. CN 103 056 646 A discloses a robot for tightening screws that comprises a fixed seat, two rotary joints, two arms, an installing plate, an air cylinder, a connecting plate and a screw-fixing machine. CN 105 232 153 A discloses a mechanical arm for minimally invasive surgery. JP H08 318483 A discloses an industrial robot for performing a linear movement. JP S61 820 A discloses an equilibrium control device for controlling the movement of inertia of a rotating member while holding an object to be conveyed. JP H09 285989 A, CN 104 942 826 A, WO 2010/130817 A1, WO 2015/142786 A1 provide further prior art.

However, the robotic arms used in current minimally invasive surgical robots are associated with some drawbacks. Specifically, conventional robotic arms are often complicated structures which are bulky and inconvenient to manipulation. More specifically, there are two major issues in using such a conventional robotic arm:
1) A horizontal movement of the conventional robotic arm is usually achieved by the rotary joint, and it relies on a number of rotary joints nested within one another, instead of a horizontal movement joint, to achieve the horizontal movement. The rotary joints together with a vertical movement joint can achieve movement of the robotic arm in the three-dimensional space. This structure of joints, on the one hand, lead to expand the dimensions of the robotic arm and increase the weight of the robotic arm and hence the overall weight of the medical surgical robot, which contravenes the compactness and lightweight requirements of medical surgical robots. On the other hand, the inter-nested rotary joints of this structure increase the arm's structural complexity and hinder its precise motion control. Moreover, the rotary joints tend to experience displacements in irrelevant directions, necessitating too much unnecessary, tedious motion adjustment.
2) Different from most industrial robotic arms which use motor-driven joints for convenient control and operation, joints in medical robotic arms are rarely motor-driven ones, because such robotic arms are often required to be "self-locked" during surgical procedures, while current motors are inevitably associated with a certain degree of risk of failure. For this reason, most robotic arms of the medical surgical robot are manipulated manually for the sake of higher reliability. However, the greatest difficulty in such manual manipulation is how to overcome, during vertical movement of the robotic arm, the gravity of an article hung on the end of the robotic arm. The current solution for this problem is to dispose the vertical movement joint on the front of the robotic arm and balance the gravity with a heavy mass. This, however, significantly increases the weight of the surgical robot and makes it difficult to guarantee precise vertical adjustment.

### Summary of the Invention

It is an objective of the present invention to provide a surgical robot and a robotic arm therefor, in order to solve one or more of the above problems with conventional robotic arms, such as high structural complexity, bulkiness and operational

To this end, the provided robotic arm includes:
a vertical movement joint, configured to move a heavy mass in a vertical direction;
a rotary joint, sleeved over a rotational shaft and configured to rotate about the rotational shaft to drive the vertical movement joint to rotate; and
a horizontal movement joint, configured to drive the rotational shaft to move in a horizontal direction.

In the robotic arm, the horizontal movement joint is fixedly connected to one end of the rotational shaft and is perpendicular to the rotational shaft, wherein the robotic arm for a surgical robot further comprises a gravity-balancing mechanism for gravitationally balancing the heavy mass hung on the vertical movement joint.

In the robotic arm, the rotary joint includes a first rotary joint and a second rotary joint, the rotational shaft including a first rotational shaft and a second rotational shaft parallel to the first rotational shaft, the first rotary joint sleeved over the first rotational shaft and able to rotate about the first rotational shaft, the second rotary joint sleeved over the second rotational shaft and able to rotate about the second rotational shaft, the connecting lever fixedly connected to both the first rotary joint and the second rotary joint, the horizontal movement joint fixedly connected to one end of the first rotational shaft, the vertical movement joint fixedly connected to one end of the second rotational shaft.

In the robotic arm, the gravity-balancing mechanism is connected to the vertical movement joint and housed in the connecting lever.

In the robotic arm, the gravity-balancing mechanism includes a constant-force spring, a spring connector, a guide assembly and a flexible member, the constant-force spring having one end fixedly connected to the connecting lever and the other end fixedly connected to the spring connector, the spring connector fixedly connected to the flexible member, the guide assembly provided at least on the connecting lever and on the vertical movement joint, the flexible member configured to be guided by the guide assembly into fixed connection with the vertical movement joint.

Preferably, in the robotic arm, the gravity-balancing mechanism may further include a sliding slot oriented in the horizontal direction, the spring connector being received in the sliding slot to guide the movement of the spring connector in the horizontal direction.

Preferably, in the robotic arm, the gravity-balancing mechanism may further include a proportional pulley, the flexible member including a first flexible member and a second flexible member, the first flexible member including a first end and a first body extending from the first end, the first end connected to the spring connector, the first body guided by the guide assembly into fixed connection with the proportional pulley, the second flexible member including a second end and a second body extending from the second end, the second end fixedly connected to the proportional pulley, the second body guided by the guide assembly into fixed connection with the vertical movement joint.

Preferably, in the robotic arm, the guide assembly may include a plurality of fixed pulleys separately disposed on the connecting lever and on the vertical movement joint.

Preferably, in the robotic arm, the vertical movement joint may include a frame, a vertical guide track fixed to the frame, and a connecting member slidably disposed on the vertical guide track, the frame in fixed connection with a connecting shaft of the second rotary joint, the connecting shaft in fixed connection with one end of the second rotational shaft.

Preferably, in the robotic arm, the frame may have a surface that is complementary to an outer circumferential surface of the second rotary joint.

Preferably, in the robotic arm, the frame may be fixedly connected to the connecting shaft by means of a mounting slot in a surface of the frame proximate the second rotary joint.

Preferably, in the robotic arm, the frame may be in the form of sleeve, the vertical guide track being housed in the frame.

Preferably, in the robotic arm, the robotic arm may further include at least one brake that is provided in the first rotary joint and/or the second rotary joint and configured to control the rotary of the first rotary joint and/or the second rotary joint on the respective rotational shaft(s).

Preferably, in the robotic arm, the horizontal movement joint may include a plurality of horizontal movement blocks that are stacked one above another and slidably connected to one another, each of the plurality of horizontal movement blocks configured to move in the horizontal direction, and wherein the rotational shaft is fixedly connected to a topmost one of the plurality of horizontal movement blocks.

Preferably, in the robotic arm, each of the horizontal movement blocks may include a base, a horizontal guide track fixed to the base, and a slider slidably disposed on the horizontal guide track, wherein for every adjacent two of the plurality of horizontal movement blocks, the base of an upper one is fixed to the slider of a lower one, and wherein the base or slider of the topmost one of the plurality of horizontal movement blocks is fixedly connected to the rotational shaft.

Preferably, in the robotic arm, two horizontal movement blocks including a first horizontal movement block and a second horizontal movement block are provided, the first horizontal movement block including a first base, a first horizontal guide track fixed to the first base and a first slider slidably disposed on the first horizontal guide track, the second horizontal movement block including a second base fixedly connected to the first slider, a second horizontal guide track fixed to the second base and a second slider slidably disposed on the second horizontal guide track.

Preferably, the robotic arm may further include a brake which is provided on the horizontal movement joint, wherein the brake is configured to limit movement of the horizontal movement joint.

The present invention also provides a surgical robot including the robotic arm as defined in any one of the above paragraphs.

In summary, the surgical robot and the robotic arm of the present invention offer the following benefits:
1. Only one horizontal movement joint is used in place of the conventional plurality of inter-nested rotary joints to enable horizontal movement of the robotic arm. Such a reduction in the number of the used joints simplifies the structure of the robotic arm, minimizes its dimensions and reduces its overall weight. In addition to the structural simplification, the motion control precision is also increased without irrelevant displacements, making simpler operation possible.
2. The first and second rotary joints are sleeved over and able to rotate about the respective parallel rotational shafts and are both fixedly connected to the connecting lever. In this way, adjustment efficiency and precision can be ensured.
3. The gravity-balancing mechanism for gravitationally balancing the vertical movement joint is received within the connecting lever, so that it enables rational internal space utilization to result in effective reductions in the robotic arm's dimensions, while fulfilling its function without affecting any other component. Moreover, it will not increase the weight of the vertical movement joint, ensuring good vertical adjustment precision thereof.
4. The frame of the vertical movement joint has a surface that is complementary to the outer circumferential surface of the second rotary joint. This makes the vertical movement joint possible to snuggly fit the second rotary joint, resulting in enhanced structural compactness.

### Brief Description of The Drawings

Fig. 1 is an overall schematic illustration of a robotic arm of a surgical robot according to an embodiment of the present invention.
Fig. 2 schematically shows movement directions of various joints in the robotic arm of Fig. 1.
Fig. 3 is a sectional view of a first rotary joint and a second rotary joint according to an embodiment of the present invention.
Fig. 4 is a front view of a vertical movement joint according to an embodiment of the present invention.
Fig. 5 is a rear view of a vertical movement joint according to an embodiment of the present invention.
Fig. 6 is a schematic illustration of a gravity-balancing mechanism according to an embodiment of the present invention.
Fig. 7 is a schematic illustration of a horizontal movement joint according to an embodiment of the present invention.
Fig. 8 is a schematic illustration of a frame of a vertical movement joint, which is in the form of a sleeve, according to an embodiment of the present invention.

In these figures,
1-horizontal movement joint; 101-first base; 102-first horizontal guide track; 103-first slider; 104-second base; 105-second horizontal guide track; 106-second slider; 107-third base; 2-first rotary joint; 3-connecting lever; 4-second rotary joint; 5-vertical movement joint; 501-base; 502-vertical guide track; 503-connecting member; 504-mounting slot; 6-first rotational shaft; 7-second rotational shaft; 8-connecting shaft; 9-first brake; 10-second brake; 11-third brake; 1201-constant-force spring; 1202-spring connector; 1203-fixed pulley; 1204-sliding slot; 13-fourth brake.

### Detailed Description of Preferred Embodiments

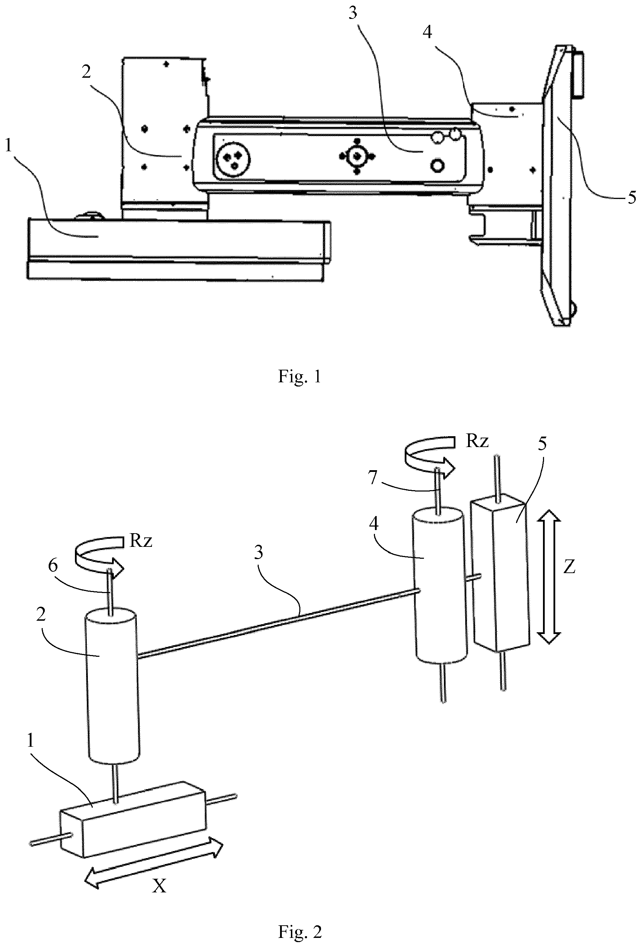

The surgical robot and the robotic arm therefor proposed in the present invention will be described in further detail below with reference to Figs. 1 to 8, so that objects, advantages and features of the invention will appear more apparent. It is noted that the figures are provided in a very simplified form not necessarily presented to scale, with the only intention to facilitate convenience and clarity in explaining a few embodiments of the invention.

Fig. 1 is an overall schematic illustration of the robotic arm of the surgical robot according to an embodiment of the present invention. Fig. 2 schematically shows movement directions of joints in the robotic arm of Fig. 1.

As shown in Figs. 1 to 2, the robotic arm is suitable for use in a surgical robot, especially in a minimally invasive surgical robot. The robotic arm is movable in the three-dimensional space and includes a horizontal movement joint 1, a first rotary joint 2, a connecting lever 3, a second rotary joint 4 and a vertical movement joint 5. The first rotary joint 2 is sleeved over a first rotational shaft 6 and is rotatable about the first rotational shaft 6. The first rotational shaft 6 may be fixed at the horizontal movement joint 1 in such a manner that it is perpendicular to the horizontal movement joint 1. The second rotary joint 4 is sleeved over a second rotational shaft 7 and is rotatable about the second rotational shaft 7. The horizontal movement joint 1 is configured to move horizontally and the vertical movement joint 5 to move vertically. Moreover, the axis of the first rotational shaft 6 is parallel to the axis of the second rotational shaft 7.

According to the present invention, only one horizontal movement joint 1 is used in place of the conventional plurality of inter-nested rotary joints to enable horizontal movement of the robotic arm. This reduction in the number of the used joints simplifies the structure of the robotic arm, minimizes its dimensions and reduces its overall weight, thus complying with the compactness and lightweight requirements of medical surgical robots. In addition to the structural simplification, the motion control precision is increased without displacements of rotary joints in irrelevant directions. Therefore, simpler operation is made possible.

It is to be noted that the number of the rotary joints is not limited to two, and in some embodiments (not shown), there may be only one rotary joint. In this case, this one rotary joint is sleeved over one rotational shaft and is able to rotate about the rotational shaft. The connecting lever 3 is fixedly connected both to the rotary joint and the vertical movement joint 5, and the horizontal movement joint 1 is fixedly connected to one end of the rotational shaft in such a manner that it is perpendicular to the rotational shaft. This also allows movement of the robotic arm in the three-dimensional space and can, of course, have the same beneficial effects as described above.

Compared to the embodiments with only one rotary joint, the embodiment of using the first rotary joint 2 and the second rotary joint 4 makes it possible to perform redundant adjustments of the robotic arm, i.e., coarse adjustments by the first rotary joint 2 and fine adjustments by the second rotary joint 4 if necessary, thereby ensuring high adjustment precision and efficiency.

Preferably, the robotic arm further includes a gravity-balancing mechanism that is connected to the vertical movement joint 5 and configured to gravitationally balance the vertical movement joint 5, i.e., balancing a heavy mass hung on the vertical movement joint 5. Moreover, the gravity-balancing mechanism is disposed within the connecting lever 3. In other embodiments, the vertical movement joint 5 may be equipped with a gravity-balancing mechanism itself. However, the vertical movement joint 5 equipping with the gravity-balancing mechanism will increase the gravitational load on the robotic arm end and affect the vertical adjustment precision. Therefore, it is preferred that the gravity-balancing mechanism is disposed within the connecting lever 3, because this can effectively reduce the dimensions of the robotic arm while enabling the mechanism to fulfill its function without affecting other components.

In the following description, the horizontal direction is referred as the "X-direction" (parallel to movement direction of the horizontal movement joint 1) and the vertical direction as the "Z-direction" (parallel to movement direction of the vertical movement joint 5). Of course, the horizontal direction may also be referred as the "Y-direction", depending on the direction in which the horizontal movement joint 1 is moving.

More preferably, the horizontal movement joint 1 and the vertical movement joint 5 are manually driven to move. Herein, the term "manually" refers to the so-called "passive adjustments" in the art. A passive adjustment of a joint refers to movement of the joint caused by a force exerted by a human being rather than by a motor or other driving means. This is because the robotic arm is required to be "self-locked" during a surgical procedure, while maintaining it in the self-locked state with a motor or the like is not reliable due to its unavoidable risk of failure.

As noted above, in case the robotic arm has two rotary joints, the robotic arm according to the embodiment has the four degrees of freedom. So it can translate in the X-direction, translate in the Z-direction, rotate about the first rotational shaft 6 and rotate about the second rotational shaft 7.

Preferably, the movement of the robotic arm of the present invention in these four degrees of freedom includes:
1) X-directional translation: translation of the robotic arm along the X-direction resulting from X-directional movement of the horizontal movement joint 1 caused by a manual driving;
2) Z-directional translation: translation of the robotic arm along the Z-direction resulting from Z-directional movement of the vertical movement joint 5 caused by a manual driving;
3) Rotation of the first rotary joint 2: the first rotary joint 2 rotates about the first rotational shaft 6 due to the fixed connection at one end of the first rotational shaft 6 with the horizontal movement joint 1, optionally, the first rotary joint 2 is in a rotatable connection with the first rotational shaft 6 by a bearing, driven either manually or by a motor, without limitation to the present invention;

It will be appreciated that since the second rotary joint 4 is integrally connected to the first rotary joint 2 by the connecting lever 3, the second rotary joint 4 will rotate in synchronization with the first rotary joint 2.

4) Rotation of the second rotary joint 4: similarly, the second rotary joint 4 rotates about the second rotational shaft 7. For example, a rotatable connection may be established with a bearing between the second rotary joint 4 and the second rotational shaft 7. As a result, the second rotary joint 4 may be driven either manually or by a motor to rotate about the second rotational shaft 7.

In this embodiment, the vertical movement joint 5 may be fixedly connected to a connecting shaft 8 proximate the second rotary joint 4. The connecting shaft 8 may be fixedly connected to one end of the second rotational shaft 7. As such, under the actuation of the second rotational shaft 7, the vertical movement joint 5 will rotate with the connecting shaft 8 proximate the second rotary joint 4.

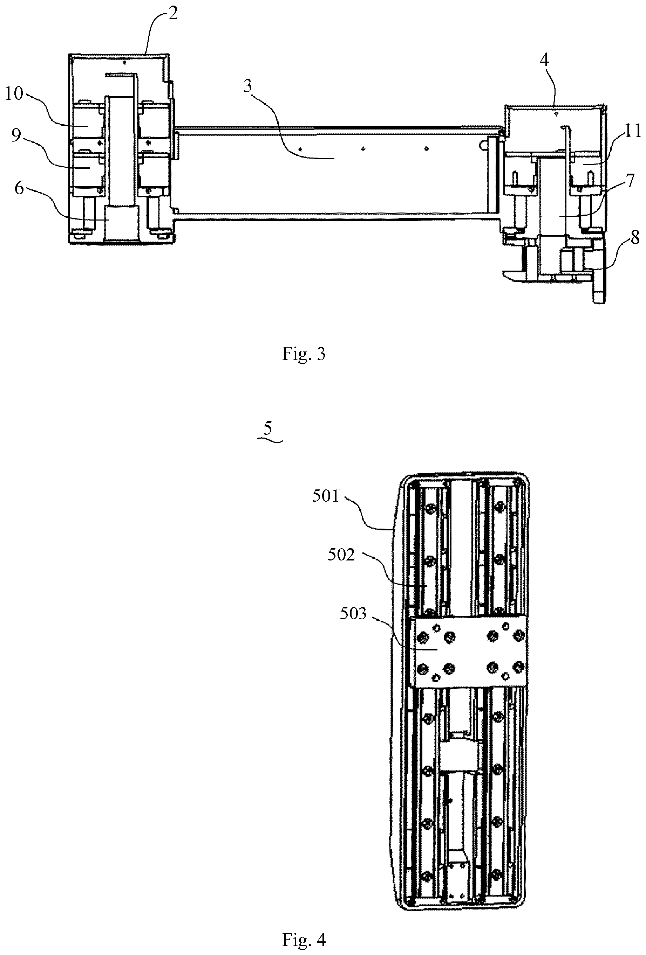

Fig. 3 is a sectional view of the first rotary joint and the second rotary joint according to an embodiment of the present invention. The vertical movement joint 5 may be connected at its back side (i.e., the side close to the second rotary joint 4) to the connecting shaft 8.

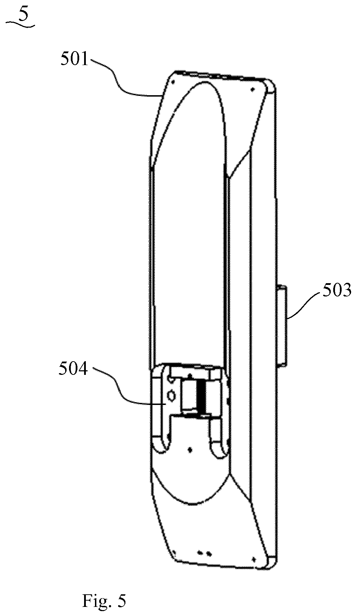

Fig. 4 is a front view of the vertical movement joint according to an embodiment of the present invention, and Fig. 5 is a rear view of the vertical movement joint of Fig. 4. Here, the front side of the vertical movement joint refers to the side thereof away from the second rotary joint 4, while the back side of the vertical movement joint refers to the side thereof proximate the second rotary joint 4.

With reference to Figs. 4 and 5, the vertical movement joint 5 may include a frame 501, a vertical guide track 502 (oriented vertically) and a connecting member 503. The back side of the frame 501 is connected to the connecting shaft 8, and the vertical guide track 502 is oriented in the vertical direction and fixed to the front side of the frame 501. The connecting member 503 is slidably connected to the vertical guide track 502 so that it can slide up and down on the vertical guide track 502. The connecting member is configured to connect an operating end portion of the robotic arm, on which a heavy mass can be hung.

In this embodiment, the frame 501 may be fixedly connected to the connecting shaft 8 by means of a mounting slot 504 in its back side (i.e., the side proximate the second rotary joint 4). Optionally, an axis of the connecting shaft 8 may be perpendicular to the second rotational shaft 7. Preferably, the back side of the frame 501 is complementary to an outer circumferential surface of the second rotary joint 4. For example, the outer circumferential surface of the second rotary joint 4 is a convexly-curved surface, whilst the back side of the frame 501 is a concavely-curved surface well matched with the outer circumferential surface of the second rotary joint 4. This can increase the firmness of the connection between these joints.

With continued reference to Fig. 3, the robotic arm may further include a first brake 9 and a second brake 10. The first brake 9 and the second brake 10 are separately housed within the first rotary joint 2 either on the same side or on the opposite sides of the first rotational shaft 6. The brakes are configured to control the rotary of the first rotary joint 2. That is, when the brakes are active, the first rotary joint 2 will be locked with respect to the first rotational shaft 6, without any relative rotation between them. When the brakes are deactivated, the first rotary joint 2 can rotate on the first rotational shaft 6 freely. The present invention is not limited to any particular number of brakes for control the rotary of the first rotary joint 2.

Correspondingly, the robotic arm may further include a third brake 11. The third brake 11 is disposed within the second rotary joint 4 on one side of the second rotational shaft 7 and is configured to control the rotary of the second rotary joint 4. The third brake 11 works in the same way as the first and second brakes 9, 10 and will not be described in further detail herein.

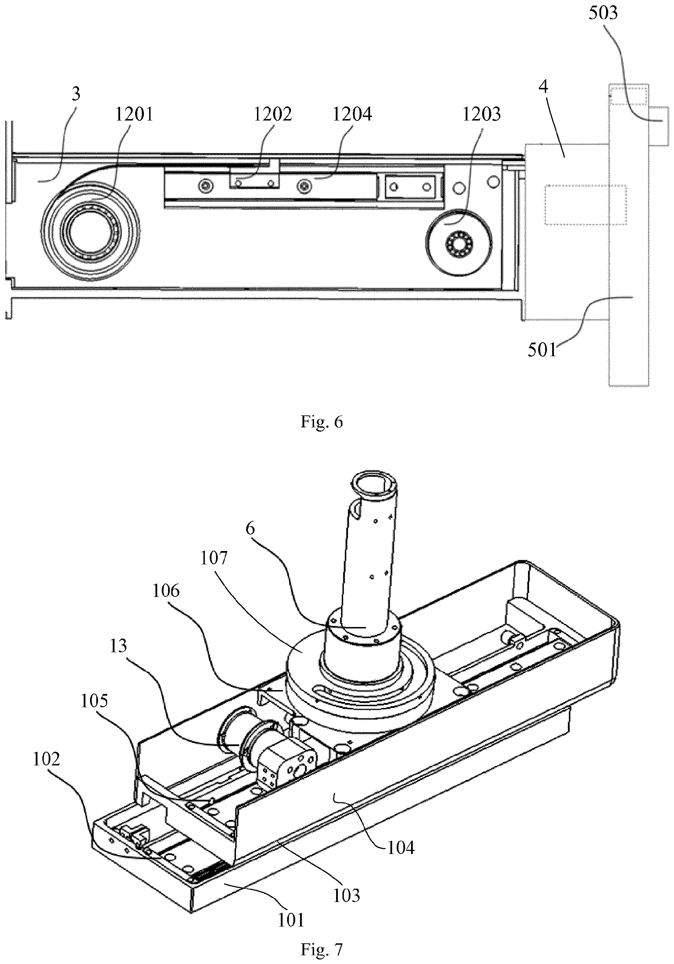

Fig. 6 is a schematic illustration of the gravity-balancing mechanism according to an embodiment of the present invention. As shown in Fig. 6, the gravity-balancing mechanism is housed in the connecting lever 3 and may include a constant-force spring 1201, a spring connector 1202, a guide assembly and a first flexible member. One end of the constant-force spring 1201 is fixed to the connecting lever 3, and the other end of the constant-force spring 1201 is connected to the spring connector 1202. The spring connector 1202 is connected to the first flexible member, and the guide assembly is provided at least on the connecting lever 3 and on the vertical movement joint 5 in order to guide an extension direction of the first flexible member so that the first flexible member is fixedly connected to the connecting member 503 of the vertical movement joint 5. The first flexible member may be, for example, a wire, a rope, a belt or the like configured to output a spring force.

Optionally, the guide assembly may include a plurality of fixed pulleys 1203. The plurality of fixed pulleys 1203 are separately disposed on the connecting lever 3 and the vertical movement joint 5 and configured to divert the first flexible member into fixed connection with the connecting member 503.

According to the present invention, the constant-force spring 1201 working as output a constant force of the gravity-balancing mechanism can achieve good gravitational balancing and avoid cumbersome calculations for dealing with gravitational changes, thus allowing a simpler gravitational balancing operation.

More preferably, the gravity-balancing mechanism may further include a sliding slot 1204 disposed within the connecting lever 3 and oriented horizontally. The spring connector 1202 may be received in the sliding slot 1204 so that the spring connector 1202 will move only in the horizontal direction with minor friction, resulting in a further improvement in gravitational balancing.

Further, the gravity-balancing mechanism may include a proportional pulley which has an inner rim and an outer rim, which a radius of the inner rim is proportional to a radius of the outer rim (not shown), and the first flexible member may include a first flexible portion and a second flexible portion. The first flexible portion has a first end and a first body extending from the first end. The first end is connected to the spring connector 1202, and the first body is guided by a corresponding one of the fixed pulleys 1203 into fixed connection with the inner rim of the proportional pulley. The second flexible portion has a second end and a second body extending from the second end. The second end is fixedly connected to the outer rim of the proportional pulley, and the second body is guided by the another corresponding one of the fixed pulleys 1203 into fixed connection with the connecting member 503. More preferably, the fixed pulley 1203 for guiding the extension direction of the first flexible portion is horizontally align to the spring connector 1202, in order to ensure a constant force output of the constant-force spring 1201. The proportional pulley may be disposed under the fixed pulley 1203 guiding the first flexible member, in order to achieve effective utilization of the internal space of the connecting lever 3.

Fig. 7 is a schematic illustration of the horizontal movement joint according to an embodiment of the present invention. As shown in Fig. 7, the horizontal movement joint 1 may include a plurality of horizontal movement blocks that are stacked one above another and slidably connected to one another. Each of the horizontal movement blocks is configured to translate in the X-direction, and the topmost one of them is connected to the first rotational shaft 6. In other words, every adjacent two of the horizontal movement blocks are slidable interconnected to each other so that the upper horizontal movement block can slide on the lower horizontal movement block. Compared to a single sliding pair, these tandem sliding pairs can lengthen the stroke of the horizontal movement joint 1, making the robotic arm movable in a greater range with higher adjustment efficiency. In addition, the horizontal dimension of the horizontal movement joint 1 can be reduced, reducing its installation space.

In this embodiment, each of the horizontal movement blocks may include a base, a horizontal guide track (oriented horizontally) and a slider. The horizontal guide track is fixed to the base, and the slider is slidable connected to the horizontal guide track so that it is horizontal slidable on the horizontal guide track. In addition, for every adjacent two of the horizontal movement blocks, the base of the upper one is fixed to the slider of the lower one. Further, the slider of, or the base covers the slider of, the topmost horizontal movement block is fixedly connected to the first rotational shaft 6.

For example, two horizontal movement blocks may be provided, as shown in Fig. 7, i.e., a first horizontal movement block and a second horizontal movement block. The first horizontal movement block includes a first base 101, a first horizontal guide track 102 fixed to the first base 101, and a first slider 103 slidably disposed on the first horizontal guide track 102. The second horizontal movement block includes a second base 104 fixed to the first slider 103, a second horizontal guide track 105 fixed to the second base 104, and a second slider 106 slidably disposed on the second horizontal guide track 105. The first rotational shaft 6 is fixedly connected to the second slider 106. Alternatively, a third base 107 may be fixedly provided on the second slider 106, and the first rotational shaft 6 is fixedly connected to the third base 107.

It will be appreciated that, when the first slider 103 is manually driven, the second base 104 will translate on the first base 101. Similarly, when the second slider 106 is manually driven, the third base 107 will translate on the second base 104. Here, those skilled in the art will recognize that the first base 101 may be fixed to any suitable surface and maintained stationary thereon. The second base 104 is able to be driven to move only by the first slider 103, and the third base 107 is able to be driven to move either by the first slider 103 or by the second slider 106.

In some other embodiments, the number of the horizontal movement blocks may also be more than two, for example three or more, with limitation to the present invention. According to the present invention, using the guide tracks and sliders to enable the horizontal movement joint 1 provides for structural simplicity, high movement reliability and convenient manipulation.

In this embodiment, the robotic arm may further include a fourth brake 13 which is provided on the horizontal movement joint 1. The fourth brake 13 is configured to control the movement of the horizontal movement joint 1. For example, the fourth brake 13 may be connected to the horizontal movement joint 1 by a second flexible member in a closed-loop manner, in order to control the position of the horizontal movement joint 1 during its movement. Specifically, when the fourth brake 13 is out of service, the horizontal movement joint 1 can move in the X-direction under the action of an external force. Upon the horizontal movement joint 1 reaching a desired position, the fourth brake 13 may be activated to prevent the horizontal movement joint 1 moving.

In case of the horizontal movement joint 1 including a plurality of horizontal movement blocks, the fourth brake 13 may be provided on the topmost horizontal movement block and the fourth brake 13 may be connected to both ends of the bottommost horizontal movement block by the second flexible member in a closed-loop manner. For example, the fourth brake 13 may be disposed on the third base 107, with the second connecting flexible member wound on the fourth brake 13 and having a left portion fixed to one end of the first base 101 and a right portion fixed to the other end of the first base 101, such that the length of the left portion and the right portion can be controlled by the fourth brake. Of course, in order for better transmission control to be achieved, the horizontal movement joint 1 may also be provided with a fixed pulley block (including a plurality of fixed pulleys) which guides the left portion into fixed connection with the end of the first base 101 and guides the right portion into fixed connection with the other end of the first base 101.

Herein, the fourth brake 13 is provided to control the movement of the horizontal movement joint 1 with the present of the second flexible member.

In some other embodiments (not shown), the horizontal movement joint 1 may alternatively include only one horizontal movement block. In this case, the horizontal movement block includes a first base 101, a first horizontal guide track 102 fixed to the first base 101, a first slider 103 slidably disposed on the first horizontal guide track 102 and a second base 104 fixed to the first slider 103. Additionally, the first rotational shaft 6 is coupled to the second base 104, and the fourth brake 13 is provided on the second base 104 or on the first slider 103 in order to prevent the movement of the first slider 103.

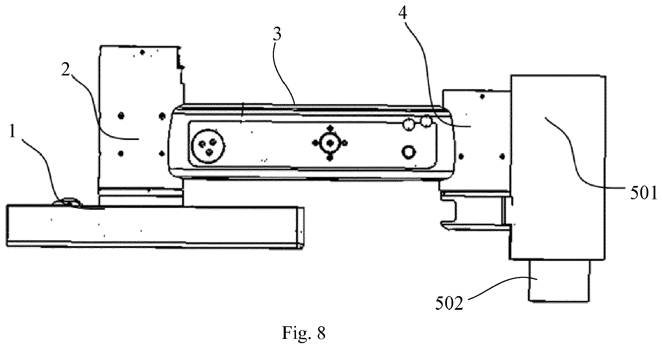

Fig. 8 schematically illustrates the base of the vertical movement joint, which is in the form of a sleeve, in accordance with an embodiment of the present invention. As shown in Fig. 8, the frame 501 of the vertical movement joint 5 has a shape of sleeve, and the vertical guide track 502 of the vertical movement joint 5 is disposed within the frame 501, thereby the vertical guide track 502 works under a desirable environment for normal operation.

In summary, according to the present invention, only one horizontal movement joint 1 is used in lieu of the conventional plurality of inter-nested rotary joints to enable horizontal movement of the robotic arm. This reduction in the number of the used joints simplifies the structure of the robotic arm, minimizes its size and reduces its overall weight. Because of the simply structure, the motion control precision is also increased without irrelevant displacements, and the robotic arm is more conveniently manipulated.

Additionally, the first and second rotary joints 2, 4 are sleeved over and able to rotate about the respective parallel axis of the rotational shafts and are both fixedly connected to the connecting lever 3. In this way, it enable that the robotic arm is adjusted efficiently and preciously.

Further, the gravity-balancing mechanism for gravitationally balancing the vertical movement joint 5 is provided within the connecting lever 3. As such, it can enable rational internal space utilization which results in effective reductions in the robotic arm's dimensions while fulfilling its function without affecting any other joint work. Moreover, it will not increase the weight of the vertical movement joint 5, with more precious vertical adjustment thereof.

Furthermore, the frame 501 of the vertical movement joint 5 has a surface that is complementary to the outer circumferential surface of the second rotary joint 4. This makes the vertical movement joint 5 possible to snuggly fit the second rotary joint 4, so that the robotic arm rotates smoothly and has a compact structure.

## Claims

1. A robotic arm for a surgical robot, comprising:
a vertical movement joint (5), configured to move a heavy mass in a vertical direction;
a rotary joint, sleeved over a rotational shaft and configured to rotate about the rotational shaft to drive the vertical movement joint (5) to rotate; and
a horizontal movement joint (1), configured to drive the rotational shaft to move in a horizontal direction,
wherein the horizontal movement joint (1) is fixedly connected to one end of the rotational shaft and is perpendicular to the rotational shaft,
wherein the robotic arm for a surgical robot further comprises a gravity-balancing mechanism for gravitationally balancing the heavy mass hung on the vertical movement joint (5), wherein
the rotary joint comprises a first rotary joint (2) and a second rotary joint (4), the rotational shaft comprising a first rotational shaft (6) and a second rotational shaft (7) parallel to the first rotational shaft (6);
the first rotary joint (2) sleeved over the first rotational shaft (6) and able to rotate about the first rotational shaft (6), the second rotary joint (4) sleeved over the second rotational shaft (7) and able to rotate about the second rotational shaft (7);
a connecting lever (3) fixedly connected to both the first rotary joint (2) and the second rotary joint (4);
the horizontal movement joint (1) fixedly connected to one end of the first rotational shaft (6), the vertical movement joint (5) fixedly connected to one end of the second rotational shaft (7),
the gravity-balancing mechanism is connected to the vertical movement joint (5) and housed in the connecting lever (3),
the gravity-balancing mechanism comprises a constant-force spring (1201), a spring connector (1202), a guide assembly and a first flexible member, the constant-force spring (1201) having one end fixedly connected to the connecting lever (3) and the other end fixedly connected to the spring connector (1202), the spring connector (1202) fixedly connected to the first flexible member, the guide assembly provided at least on the connecting lever (3) and on the vertical movement joint (5), the first flexible member configured to be guided by the guide assembly into fixed connection with the vertical movement joint (5).

2. The robotic arm for a surgical robot of claim 1, wherein the gravity-balancing mechanism further comprises a sliding slot (1204) oriented in the horizontal direction, the spring connector (1202) being received in the sliding slot (1204) to stop movement of the spring connector (1202) in the horizontal direction.

3. The robotic arm for a surgical robot of claim 1 or 2, wherein the gravity-balancing mechanism further comprises a proportional pulley, the first flexible member comprising a first flexible portion and a second flexible portion, the first flexible portion comprising a first end and a first body extending from the first end, the first end connected to the spring connector (1202), the first body guided by the guide assembly into fixed connection with the proportional pulley, the second flexible portion comprising a second end and a second body extending from the second end, the second end fixedly connected to the proportional pulley, the second body guided by the guide assembly into fixed connection with the vertical movement joint (5).

4. The robotic arm for a surgical robot of claim 3, wherein the guide assembly comprises a plurality of fixed pulleys (1203) separately disposed on the connecting lever (3) and on the vertical movement joint (5).

5. The robotic arm for a surgical robot of claim 1, wherein the vertical movement joint (5) comprises a frame, a vertical guide track (502) fixed to the frame, and a connecting member (503) slidably disposed on the vertical guide track (502), the frame in fixed connection with a connecting shaft (8) proximate the second rotary joint (4), the connecting shaft (8) in fixed connection with one end of the second rotational shaft (7).

6. The robotic arm for a surgical robot of claim 5, wherein the frame has a surface that is complementary to an outer circumferential surface of the second rotary joint (4).

7. The robotic arm for a surgical robot of claim 5 or 6, wherein the frame is fixedly connected to the connecting shaft by means of a mounting slot in a surface of the frame proximate the second rotary joint.

8. The robotic arm for a surgical robot of claim 5 or 6, wherein the frame is in the form of sleeve, the vertical guide track being housed in the frame.

9. The robotic arm for a surgical robot of claim 1, further comprising at least one brake that is provided in the first rotary joint (2) and/or the second rotary joint (4) and configured to lock and immobilize the first rotary joint (2) and/or the second rotary joint (4) onto the respective rotational shaft(s).

10. The robotic arm for a surgical robot of claim 1, wherein the horizontal movement joint (1) comprises a plurality of horizontal movement blocks that are stacked one above another and slidably connected to one another, each of the plurality of horizontal movement blocks configured to move in the horizontal direction, and wherein the rotational shaft is fixedly connected to a topmost one of the plurality of horizontal movement blocks.

11. The robotic arm for a surgical robot of claim 10, wherein each of the plurality of horizontal movement blocks comprises a base (501), a horizontal guide track fixed to the base (501), and a slider slidably disposed on the horizontal guide track, wherein for every adjacent two of the plurality of horizontal movement blocks, the base (501) of an upper one is fixed to the slider of a lower one, and wherein the slider of, or the base (501) which covers the slider of, the topmost one of the plurality of horizontal movement blocks is fixedly connected to the rotational shaft.

12. The robotic arm for a surgical robot of claim 11, wherein two horizontal movement blocks comprising a first horizontal movement block and a second horizontal movement block are provided, the first horizontal movement block comprising a first base (101), a first horizontal guide track (102) fixed to the first base (101) and a first slider (103) slidably disposed on the first horizontal guide track (102), the second horizontal movement block comprising a second base (104) fixedly connected to the first slider (103), a second horizontal guide track (105) fixed to the second base (104) and a second slider (106) slidably disposed on the second horizontal guide track (105).

13. The robotic arm for a surgical robot of any one of claims 10 to 12, further comprising a brake which is provided on the horizontal movement joint (1), wherein the brake is configured to stop movement of the horizontal movement joint (1).

14. A surgical robot, comprising the robotic arm of any one of claims 1 to 13.

## Patentansprüche

1. Roboterarm für einen chirurgischen Roboter, umfassend:
ein Vertikalbewegungsgelenk (5), das ausgebildet ist, eine schwere Masse in einer vertikalen Richtung zu bewegen;
ein Rotationsgelenk, welches über eine Rotationswelle geschoben und ausgebildet ist, um die Rotationswelle zu rotieren, um das Vertikalbewegungsgelenk (5) zur Rotation anzutreiben; und
ein Horizontalbewegungsgelenk (1), das zum Antreiben der Rotationswelle zur Bewegung in einer horizontalen Richtung ausgebildet ist,
wobei das Horizontalbewegungsgelenk (1) fest mit einem Ende der Rotationswelle verbunden ist und senkrecht zu der Rotationswelle ist,
wobei der Roboterarm für einen chirurgischen Roboter ferner einen Gravitationsausgleichsmechanismus zum gravitativen Ausgleichen der schweren Masse umfasst, die an dem Vertikalbewegungsgelenk (5) aufgehängt ist, wobei
das Rotationsgelenk ein erstes Rotationsgelenk (2) und ein zweites Rotationsgelenk (4) umfasst, wobei die Rotationswelle eine erste Rotationswelle (6) und eine zu der ersten Rotationswelle (6) parallele zweite Rotationswelle (7) umfasst;
wobei das erste Rotationsgelenk (2) über die erste Rotationswelle (6) geschoben und um die erste Rotationswelle (6) rotierbar ist, wobei das zweite Rotationsgelenk (4) über die zweiten Rotationswelle (7) geschoben und um die zweite Rotationswelle (7) rotierbar ist;
einen sowohl mit dem ersten Rotationsgelenk (2) als auch mit dem zweiten Rotationsgelenk (4) fest verbundenen Verbindungshebel (3);
wobei das Horizontalbewegungsgelenk (1) fest mit einem Ende der ersten Rotationswelle (6) verbunden ist, wobei das Vertikalbewegungsgelenk (5) fest mit einem Ende der zweiten Rotationswelle (7) verbunden ist,
wobei der Gravitationsausgleichsmechanismus mit dem Vertikalbewegungsgelenk (5) verbunden und im Verbindungshebel (3) untergebracht ist,
wobei der Gravitationsausgleichsmechanismus eine Konstantkraftfeder (1201), einen Federverbinder (1202), eine Führungsanordnung und ein erstes flexibles Element umfasst, wobei die Konstantkraftfeder (1201) ein Ende aufweist, das fest mit dem Verbindungshebel (3) verbunden ist, und wobei das andere Ende fest mit dem Federverbinder (1202) verbunden ist, wobei der Federverbinder (1202) fest mit dem ersten flexiblen Element verbunden ist, wobei die Führungsanordnung zumindest am Verbindungshebel (3) und am Vertikalbewegungsgelenk (5) bereitgestellt ist, wobei das erste flexible Element ausgebildet ist, durch die Führungsanordnung in eine feste Verbindung mit dem Vertikalbewegungsgelenk (5) geführt zu werden.

2. Roboterarm für einen chirurgischen Roboter nach Anspruch 1, wobei der Gravitationsausgleichsmechanismus ferner einen in horizontaler Richtung ausgerichteten Gleitschlitz (1204) umfasst, wobei der Federverbinder (1202) in dem Gleitschlitz (1204) zum Stoppen einer Bewegung des Federverbinders (1202) in der horizontalen Richtung aufgenommen ist.

3. Roboterarm für einen chirurgischen Roboter nach Anspruch 1 oder 2, wobei der Gravitationsausgleichsmechanismus ferner eine Proportionalriemenscheibe umfasst, wobei das erste flexible Element einen ersten flexiblen Abschnitt und einen zweiten flexiblen Abschnitt umfasst, wobei der erste flexible Abschnitt ein erstes Ende und einen ersten Körper umfasst, der sich von dem ersten Ende erstreckt, wobei das erste Ende mit dem Federverbinder (1202) verbunden ist, wobei der erste Körper durch die Führungsanordnung in eine feste Verbindung mit der Proportionalriemenscheibe geführt wird, wobei der zweite flexible Abschnitt ein zweites Ende und einen zweiten Körper umfasst, der sich von dem zweiten Ende erstreckt, wobei das zweite Ende fest mit der Proportionalriemenscheibe verbunden ist, wobei der zweite Körper durch die Führungsanordnung in eine feste Verbindung mit dem Vertikalbewegungsgelenk (5) geführt wird.

4. Roboterarm für einen chirurgischen Roboter nach Anspruch 3, wobei die Führungsanordnung eine Vielzahl feststehender Rollen (1203) umfasst, die separat an dem Verbindungshebel (3) und an dem Vertikalbewegungsgelenk (5) angeordnet sind.

5. Roboterarm für einen chirurgischen Roboter nach Anspruch 1, wobei das Vertikalbewegungsgelenk (5) einen Rahmen, eine am Rahmen befestigte Vertikalführungsbahn (502) und ein verschiebbar an der Vertikalführungsbahn (502) angeordnetes Verbindungselement (503) umfasst, wobei der Rahmen in fester Verbindung mit einer Verbindungswelle (8) in der Nähe des zweiten Rotationsgelenks (4) ist, wobei die Verbindungswelle (8) in fester Verbindung mit einem Ende der zweiten Rotationswelle (7) ist.

6. Roboterarm für einen chirurgischen Roboter nach Anspruch 5, wobei der Rahmen eine Oberfläche aufweist, die komplementär zu einer äußeren Umfangsoberfläche des zweiten Rotationsgelenks (4) ist.

7. Roboterarm für einen chirurgischen Roboter nach Anspruch 5 oder 6, wobei der Rahmen mittels eines Montageschlitzes in einer Oberfläche des Rahmens in der Nähe des zweiten Rotationsgelenks fest mit der Verbindungswelle verbunden ist.

8. Roboterarm für einen chirurgischen Roboter nach Anspruch 5 oder 6, wobei der Rahmen die Form einer Hülse hat, wobei die Vertikalführungsschiene in dem Rahmen untergebracht ist.

9. Roboterarm für einen chirurgischen Roboter nach Anspruch 1, ferner umfassend mindestens eine Bremse, die in dem ersten Rotationsgelenk (2) und/oder dem zweiten Rotationsgelenk (4) vorgesehen ist und ausgebildet ist, das erste Rotationsgelenk (2) und/oder das zweite Rotationsgelenk (4) auf die jeweilige(n) Rotationswelle(n) festzustellen und zu immobilisieren.

10. Roboterarm für einen chirurgischen Roboter nach Anspruch 1, wobei das Horizontalbewegungsgelenk (1) eine Vielzahl von Horizontalbewegungsblöcken umfasst, die übereinandergestapelt und miteinander verschiebbar verbunden sind, wobei jeder der Vielzahl von Horizontalbewegungsblöcken ausgebildet ist, sich in der horizontalen Richtung zu bewegen, und wobei die Rotationswelle fest mit einem Obersten der Vielzahl von Horizontalbewegungsblöcken verbunden ist.

11. Roboterarm für einen chirurgischen Roboter nach Anspruch 10, wobei jeder der Vielzahl von Horizontalbewegungsblöcken eine Basis (501), eine an der Basis (501) befestigte Horizontalführungsschiene und einen verschiebbar auf der Horizontalführungsschiene angeordneten Schieber umfasst, wobei für jeweils zwei benachbarte der Vielzahl von Horizontalbewegungsblöcken die Basis (501) eines Oberen an dem Schieber eines Unteren befestigt ist, und wobei der Schieber, oder die den Schieber abdeckende Basis (501), der Obersten der Vielzahl von Horizontalbewegungsblöcken fest mit der Rotationswelle verbunden ist.

12. Roboterarm für einen chirurgischen Roboter nach Anspruch 11, wobei zwei Horizontalbewegungsblöcke mit einem ersten Horizontalbewegungsblock und einem zweiten Horizontalbewegungsblock vorgesehen sind, wobei der erste Horizontalbewegungsblock eine erste Basis (101) und eine erste an der ersten Basis (101) befestigte Horizontalführungsschiene (102) und einen ersten Schieber (103) umfasst, der verschiebbar auf der ersten Horizontalführungsschiene (102) angeordnet ist, wobei der zweite Horizontalbewegungsblock eine zweite Basis (104) umfasst, die fest mit dem ersten Schieber (103) verbunden ist, eine zweite an der zweiten Basis (104) befestigte Horizontalführungsschiene (105) und einen zweiten Schieber (106) umfasst, der verschiebbar auf der zweiten Horizontalführungsschiene (105) angeordnet ist.

13. Roboterarm für einen chirurgischen Roboter nach einem der Ansprüche 10 bis 12, ferner umfassend eine Bremse, die an dem Horizontalbewegungsgelenk (1) vorgesehen ist, wobei die Bremse ausgebildet ist, eine Bewegung des Horizontalbewegungsgelenks (1) zu stoppen.

14. Chirurgischer Roboter, umfassend den Roboterarm nach einem der Ansprüche 1 bis 13.

## Revendications

1. Bras robotisé pour un robot chirurgical, comprenant :
une articulation à mouvement vertical (5), conçue pour déplacer une masse lourde dans une direction verticale ;
une articulation rotative, manchonnée sur un arbre rotatif et conçue pour tourner autour de l'arbre rotatif afin d'amener l'articulation à mouvement vertical (5) à tourner ; et
une articulation à mouvement horizontal (1), conçue pour amener l'arbre rotatif à se déplacer dans une direction horizontale,
dans lequel l'articulation à mouvement horizontal (1) est reliée à demeure à une extrémité de l'arbre rotatif et est perpendiculaire à l'arbre rotatif,
dans lequel le bras robotisé pour un robot chirurgical comprend en outre un mécanisme d'équilibrage par gravité pour équilibrer par gravité la masse lourde suspendue sur l'articulation à mouvement vertical (5), dans lequel
l'articulation rotative comprend une première articulation rotative (2) et une seconde articulation rotative (4), l'arbre rotatif comprenant un premier arbre rotatif (6) et un second arbre rotatif (7) parallèle au premier arbre rotatif (6) ;
la première articulation rotative (2) manchonnée sur le premier arbre rotatif (6) et pouvant tourner autour du premier arbre rotatif (6), la seconde articulation rotative (4) manchonnée sur le second arbre rotatif (7) et pouvant tourner autour du second arbre rotatif (7) ;
un levier de liaison (3) relié à demeure à la fois à la première articulation rotative (2) et à la seconde articulation rotative (4) ;
l'articulation à mouvement horizontal (1) reliée à demeure à une extrémité du premier arbre rotatif (6), l'articulation à mouvement vertical (5) reliée à demeure à une extrémité du second arbre rotatif (7),
le mécanisme d'équilibrage par gravité est relié à l'articulation à mouvement vertical (5) et logé dans le levier de liaison (3),
le mécanisme d'équilibrage par gravité comprend un ressort à force constante (1201), un raccord à ressort (1202), un ensemble de guidage et un premier élément flexible, le ressort à force constante (1201) ayant une extrémité reliée à demeure au levier de liaison (3) et l'autre extrémité reliée à demeure au raccord à ressort (1202), le raccord à ressort (1202) étant relié à demeure au premier élément flexible, l'ensemble de guidage étant situé au moins sur le levier de liaison (3) et sur l'articulation à mouvement vertical (5), le premier élément flexible étant conçu pour être guidé par l'ensemble de guidage en liaison fixe avec l'articulation à mouvement vertical (5).

2. Bras robotisé pour un robot chirurgical selon la revendication 1, dans lequel le mécanisme d'équilibrage par gravité comprend en outre une fente coulissante (1204) orientée dans la direction horizontale, le raccord à ressort (1202) étant reçu dans la fente coulissante (1204) pour arrêter le mouvement du raccord à ressort (1202) dans la direction horizontale.

3. Bras robotisé pour un robot chirurgical selon la revendication 1 ou 2, dans lequel le mécanisme d'équilibrage par gravité comprend en outre une poulie proportionnelle, le premier élément flexible comprenant une première partie flexible et une seconde partie flexible, la première partie flexible comprenant une première extrémité et un premier corps s'étendant depuis la première extrémité, la première extrémité étant reliée au raccord à ressort (1202), le premier corps étant guidé par l'ensemble de guidage en liaison fixe avec la poulie proportionnelle, la seconde partie flexible comprenant une seconde extrémité et un second corps s'étendant depuis la seconde extrémité, la seconde extrémité étant reliée à demeure à la poulie proportionnelle, le second corps étant guidé par l'ensemble de guidage en liaison fixe avec l'articulation à mouvement vertical (5).

4. Bras robotisé pour un robot chirurgical selon la revendication 3, dans lequel l'ensemble de guidage comprend une pluralité de poulies fixes (1203) disposées séparément sur le levier de liaison et sur l'articulation à mouvement vertical (5).

5. Bras robotisé pour un robot chirurgical selon la revendication 1, dans lequel l'articulation à mouvement vertical (5) comprend un cadre, un rail de guidage vertical (502) fixé au cadre, et un élément de liaison (503) disposé de manière coulissante sur le rail de guidage vertical (502), le cadre étant en liaison fixe avec un arbre de liaison (8) à proximité de la seconde articulation rotative (4), l'arbre de liaison (8) étant en liaison fixe avec une extrémité du second arbre rotatif (7).

6. Bras robotisé pour un robot chirurgical selon la revendication 5, dans lequel le cadre a une surface qui est complémentaire à une surface circonférentielle externe de la seconde articulation rotative (4).

7. Bras robotisé pour un robot chirurgical selon la revendication 5 ou 6, dans lequel le cadre est relié à demeure à l'arbre de liaison au moyen d'une fente de montage dans une surface du cadre à proximité de la seconde articulation rotative.

8. Bras robotisé pour un robot chirurgical selon la revendication 5 ou 6, dans lequel le cadre est sous la forme d'un manchon, le rail de guidage vertical étant logé dans le cadre.

9. Bras robotisé pour un robot chirurgical selon la revendication 1, comprenant en outre au moins un frein qui est situé dans la première articulation rotative (2) et/ou la seconde articulation rotative (4) et conçu pour verrouiller et immobiliser la première articulation rotative (2) et/ou la seconde articulation rotative sur le ou les arbres rotatifs respectifs.

10. Bras robotisé pour un robot chirurgical selon la revendication 1, dans lequel l'articulation à mouvement horizontal (1) comprend une pluralité de blocs à mouvement horizontal qui sont empilés les uns au-dessus des autres et reliés de manière coulissante les uns aux autres, chaque bloc de la pluralité de blocs à mouvement horizontal étant conçu pour se déplacer dans la direction horizontale, et dans lequel l'arbre rotatif est relié à demeure à un bloc le plus haut de la pluralité de blocs à mouvement horizontal.

11. Bras robotisé pour un robot chirurgical selon la revendication 10, dans lequel chaque bloc de la pluralité de blocs à mouvement horizontal comprend une base (501), un rail de guidage horizontal fixé à la base (501), et un curseur disposé de manière coulissante sur le rail de guidage horizontal, dans lequel pour chaque paire de blocs adjacents de la pluralité de blocs à mouvement horizontal, la base (501) d'un bloc supérieur est fixée au curseur d'un bloc inférieur, et dans lequel le curseur, ou la base (501) qui recouvre le curseur, du bloc le plus haut de la pluralité de blocs à mouvement horizontal est relié(e) à demeure à l'arbre rotatif.

12. Bras robotisé pour un robot chirurgical selon la revendication 11, dans lequel deux blocs à mouvement horizontal comprenant un premier bloc à mouvement horizontal et un second bloc à mouvement horizontal sont prévus, le premier bloc à mouvement horizontal comprenant une première base (101), un premier rail de guidage horizontal (102) fixé à la première base (101) et un premier curseur (103) disposé de manière coulissante sur le premier rail de guidage horizontal (102), le second bloc à mouvement horizontal comprenant une seconde base (104) reliée à demeure au premier curseur (103), un second rail de guidage horizontal (105) fixé à la seconde base (104) et un second curseur (106) disposé de manière coulissante sur le second rail de guidage horizontal (105).

13. Bras robotisé pour un robot chirurgical selon l'une quelconque des revendications 10 à 12, comprenant en outre un frein qui est situé sur l'articulation à mouvement horizontal (1), dans lequel le frein est conçu pour arrêter le mouvement de l'articulation à mouvement horizontal (1).

14. Robot chirurgical, comprenant le bras robotisé selon l'une quelconque des revendications 1 à 13.
